# EUROPEAN PATENT APPLICATION

(11) **EP 1 288 188 A1**
(43) Date of publication of application: **05.03.2003**
(21) Application number: 01203244.7
(22) Date of filing: 28.08.2001
(51) Int. Cl.: C07C 209/48, B01J 23/58, B01J 23/78, B01J 23/66, B01J 37/02

(54) **Supported catalyst composition, processes for the preparation thereof and use of the supported catalyst composition**

(71) Applicant: Avantium International B.V., 1014 BV Amsterdam (NL)
(72) Inventor: van den Brink, Peter John, 3972 EJ Driebergen (NL)
(74) Representative: Iemenschot, Johannes Andreas, Ir.

(57) **Abstract**

The present invention relates to a supported catalyst composition comprising:
- at least one metal selected from the group consisting of nickel, palladium, platinum, copper, silver, gold, cobalt, rhodium and iridium; and
- at least one metal selected from the group consisting of lithium, sodium, potassium, rubidium, cesium, beryllium, magnesium, calcium, strontium and barium;
wherein the composition comprises at least 5% by weight lithium, sodium, potassium, rubidium, cesium, beryllium, magnesium, calcium, strontium or barium, or a mixture of at least two thereof, based on the supported catalyst composition.

Also, the present invention relates to processes for the preparation of the supported catalyst composition and to the use of the supported catalyst composition as a hydrogenation catalyst.

## Description

The present invention relates to a supported catalyst composition, in particular for use in the hydrogenation of nitriles.

In the hydrogenation of nitriles many different, optionally supported, catalyst compositions are used, wherein generally a mixture of primary, secondary and tertiary amines is obtained, depending on the catalyst and reaction conditions used. Much research has been done on the control of the hydrogenation to one type of amine, as the isolation of the respective amines requires additional apparatus and costs additional energy.

A problem that is encountered with known hydrogenation catalysts is that a high conversion of the nitriles in combination with a high selectivity of the hydrogenation reaction is difficult to obtain.

A further problem of known hydrogenation catalysts, such as Raney-nickel, is that they are often difficult to handle, which renders their use less attractive. Moreover, environmental objections are often associated with preparing and using of the known catalysts.

Therefore, it is an object of the present invention to provide novel hydrogen catalysts showing high conversion rates in combination with high selectivity.

It is a further object of the present invention to provide novel catalysts as such.

The above objects are achieved by a supported catalyst composition according to the present invention comprising:
- at least one metal selected from the group consisting of nickel, palladium, platinum, copper, silver, gold, cobalt, rhodium and iridium; and
- at least one metal selected from the group consisting of lithium, sodium, potassium, rubidium, cesium, beryllium, magnesium, calcium, strontium and barium;
wherein the composition comprises at least 1% by weight of lithium, sodium, potassium, rubidium, cesium, beryllium, magnesium, calcium, strontium or barium, or a mixture of at least two thereof, based on the weight of the supported catalyst composition.

The supported catalyst composition according to the present invention shows surprisingly a high conversion rate and selectivity.

In particular it has been found that the supported catalyst composition according to the present invention exhibits, when used in the hydrogenation of nitriles, a surprisingly high conversion rate in combination with a high selectivity to the primary amine.

A further advantage of the catalyst of the present invention is that it can be used in a fixed bed reactor, but also in a batch process. In addition, no additional additives are required, such as ammonia.

According to a preferred embodiment of the present invention the catalyst composition comprises 4 - 20% by weight of lithium, sodium, potassium, rubidium, cesium, beryllium, magnesium, calcium, strontium or barium, or a mixture of at least two thereof, based on the weight of the supported catalyst composition.

It has been found that herewith the catalyst exhibits a particularly high selectivity and conversion. When less than 4% by weight is used, a high conversion rate may be obtained, but a high selectivity is often difficult to obtain. More than 20% by weight is not easily obtained using single step impregnation processes.

Preferably, the composition comprises at least 3 % by weight of nickel, palladium, platinum, copper, silver, gold, cobalt, rhodium and iridium.

According to a particularly preferred embodiment of the present invention, and as is shown in the examples hereinafter, the catalyst composition comprises at least 4% by weight of lithium or calcium based on the weight of the supported catalyst composition.

Further it is preferred that the catalyst composition comprises nickel or copper or a mixture thereof. Preferably, at least 3% by weight of nickel or copper is present. Especially advantageous results are obtained when the composition comprises nickel or copper and lithium. Also, as is shown in the Examples, advantageous results are obtained when the composition comprises copper and calcium, preferably about 10% by weight of calcium based on the weight of the supported catalyst composition.

In the supported catalyst composition according to the present invention any suitable support may be used. It has been surprisingly found that very good results are obtained, when the catalyst composition is supported on a porous support. Preferably the porous support comprises substantially metal oxides selected from the group consisting of silicon, aluminum, magnesium, calcium, titanium or zirconium. Preferably the support comprises alumina, more preferably gamma-alumina.

It has been found that good hydrogenation results are obtained when the catalyst comprises copper and/or nickel and lithium at concentrations ranging from 0.1 to 50 mmol metal per gram alumina. In particular suitable hydrogenation results are obtained when the catalyst comprises copper at a concentration of 0.8-0.9 mmol, preferably about 0.85 mmol copper per gram of alumina, and lithium at a concentration of 8-9, preferably about 8.1 mmol lithium per gram of alumina; or nickel at a concentration of 0.85-0.95 mmol, in particular about 0.9 mmol nickel per gram alumina, and lithium at a concentration of 8-9, preferably about 8.1 mmol lithium per gram of alumina. It has also been found that good hydrogenation results are obtained when the catalyst comprises copper and calcium at concentrations ranging from 0.1 to 50 mmol metal per gram alumina. In particular suitable hydrogenation results are obtained when the catalyst comprises copper at a concentration of 0.8-0.9 mmol; preferably about 0.85 mmol copper per gram of alumina, and calcium at a concentration of 2-3 mmol, preferably about 2.5 mmol calcium per gram alumina.

In a further aspect the present invention relates to a process for the preparation of a catalyst composition, wherein the process comprises the following steps:
(a) treating a support with a first solution containing such an amount of at least one dissolved salt of a metal selected from the group consisting of lithium, sodium, potassium, rubidium, cesium, beryllium, magnesium, calcium, strontium and barium, or a mixture of at least two thereof, that the composition obtained in step (d) comprises at least 1% by weight of lithium, sodium, potassium, rubidium, cesium, beryllium, magnesium, calcium, strontium or barium, or a mixture of at least two thereof, based on the weight of the supported catalyst composition;
(b) drying the treated support obtained in step (a);
(c) treating the dried support obtained in step (b) with a second solution containing at least one dissolved salt of a metal selected from the group consisting of nickel, palladium, platinum, copper, silver, gold, cobalt, rhodium and iridium; and
(d) drying the composition obtained in step (c).

Herewith a surprisingly simple and elegant process for obtaining supported catalyst composition is provided.

An alternative preparation route has been found where a solution of lithium nitrate and copper nitrate was impregnated onto the alumina support in one step ("co-impregnation") A solution was prepared comprising of lithium nitrate and copper nitrate. The support was treated with the solution, such that the composition obtained 5% by weight of lithium and 5% by weight of copper based on the weight of the supported catalyst composition. The treated support was dried as described in steps (b) and (d). The catalyst, having the same composition as given in example 1, shows similar activity and selectivity as the catalyst of example 1.

Therefore the present invention relates, in an alternative embodiment, to a process for the preparation of a catalyst composition, wherein the process comprises the following steps:
(x) treating a support with a first solution containing such an amount of at least one dissolved metal selected from the group consisting of lithium, sodium, potassium, rubidium, cesium, beryllium, magnesium, calcium, strontium and barium, or a mixture of at least two thereof, that the composition obtained in step (y) comprises at least 1% by weight of lithium, sodium, potassium, rubidium, cesium, beryllium, magnesium, calcium, strontium or barium, or a mixture of at least two thereof, based on the weight of the supported catalyst composition; and with a second solution containing at least one dissolved salt of a metal selected from the group consisting of nickel, palladium, platinum, copper, silver, gold, cobalt, rhodium and iridium; and
(y) drying the treated support obtained in step (x).

The person skilled in the art will understand that the support may also be treated first with the second solution. Also the support may be treated with the first and second solution at the same time.

It has been found that the catalysts obtained in the processes according to the present invention exhibit surprisingly high conversion rates, when used in the hydrogenation of nitriles, in combination with a high selectivity to the primary amine.

Also, in many known processes for preparation of catalysts batch reactors are used where the catalyst, which is usually in the form of a fine powder, needs to be separated from the product(s). Therefore additional process steps are necessary, for example product recycle steps to obtain sufficient conversion to the desired product, additional purification steps, such as distillation, the addition of other chemicals to enhance selectivity, such as the addition of NaOH or ammonia, all of which require additional processing hardware. In contrast herewith, the processes according to the present invention provide a single pass process, i.e. the product does not require a recycle.

Preferably, treating in step (a) and (c) or (x) comprises impregnation by a solution. However, the person skilled in the art will understand that any other way of treating may be performed according to the present invention.

According to a preferred embodiment of the process according to the present invention, the composition is calcined after the drying steps (b) and (d) or (y). Very good results are obtained when the composition is calcined between 350-600°C, preferably between 500-580°C, most preferably at about 550°C.

Further it is preferred that the composition is dried in steps (b) and (d) or (y) between 80-150°C, preferably between 125-145°C, most preferably at about 140°C.

Preferably, the treated support is dried in step (b) at a temperature of between 80 and 150°C for at least 0.5 h, and calcined at a temperature of between 350 to 600°C for at least 0.5 h, while the heating rate is between 30 and 300°C/h. More preferably the treated support is dried in step (b) at a temperature of about 120°C during 2 h, and calcined at about 550°C during 2 h, with a heating rate of about 120°C/h.

Preferably the composition obtained in step (c) is dried in step (d) at a temperature of between 80 and 150°C for at least 0.5 h, and calcined at a temperature of between 350 to 600°C for at least 0.5 h, while the heating rate is between 30 and 300°C/h. More preferably, the composition is dried in step (d) at a temperature of about 140°C during 2 h, and calcined at a temperature of about 550°C during 2 h, with a heating rate of about 120°C/h.

As is shown in the examples hereinafter, the first solution used in step (a) or (x) preferably contains a lithium salt, preferably lithium nitrate. Also it is preferred that the first solution in step (a) preferably contains a calcium salt, preferably calcium nitrate.

Further it is preferred that the second solution used in step (c) or (x) contains a copper salt or a nickel salt or a mixture thereof, preferably copper or nickel nitrate.

It has been found that good results are obtained when the support comprises a porous support. Preferably the support comprises alumina, preferably gamma-alumina.

Also, the present invention relates to a catalyst composition obtainable according to the process according to the present invention.

Finally, the present invention relates to the use of the catalyst composition according to the present invention, in particular as a hydrogenation catalyst.

Preferably the catalysts according to the present invention are used in the hydrogenation of nitriles, in particular in the selective hydrogenation of nitriles for the production of primary amines.

The person skilled in the art will understand that 'nitriles' comprises its normal meaning, i.e. containing a cyano group. Therefore, 'nitriles' comprises acetonitrile, adiponitrile, etc.

Further, the person skilled in the art will understand that many modifications may be made without departing from the spirit of the invention. For instance, in the hydrogenation of nitriles suitable additional compounds such as a cocatalyst may be used, if desired or appropriate.

Hereinafter the present invention will be illustrated in more detail by a series of non-limiting examples.

### Example 1

### Preparation of a copper lithium alumina catalyst (5Cu5LiALU)

A portion of 10 g gamma-alumina (AX350, CRI, Ghent, Belgium), comprising particles with an average particle size between 0.2 and 0.6 mm, and with a pore volume of 0.7 ml/g, was impregnated with 7.0 ml of a solution of 6.3 g of lithium nitrate dissolved in water. After homogenizing, the product was dried at 140°C for 2 h and subsequently calcined at 550°C for 2 h while heating at a rate of 120°C/h. A portion of 1 g of the lithium-alumina was impregnated with 0.55 ml of a solution of 0.2 g of copper(II) nitrate trihydrate in water. After homogenizing, the product was dried at 120°C for 2 h and subsequently calcined at 550°C for 2 h while heating at a rate of 60°C/h. A supported catalyst was obtained, comprising 4.5% Cu, 4.6% Li, based on the weight of the supported catalyst composition.

### Example 2

### Preparation of a nickel lithium alumina catalyst (5Ni5LiALU)

A portion of 10 g gamma-alumina (AX350, CRI, Ghent, Belgium), comprising particles with an average particle size between 0.2 and 0.6 mm, and with a pore volume of 0.7 ml/g, was impregnated with 7.0 ml of a solution of 6.3 g of lithium nitrate dissolved in water. After homogenizing, the product was dried at 140°C for 2 h and subsequently calcined at 550°C for 2 h while heating at a rate of 120°C/h. A portion of 1 g of the lithium-alumina was impregnated with 0.55 ml of a solution of 0.26 g of nickel(II) nitrate hexahydrate in water. After homogenizing, the product was dried at 120°C for 2 h and subsequently calcined at 550°C for 2 h while heating at a rate of 60°C/h. A supported catalyst was obtained, comprising 4.4% Ni, 4.6% Li, based on the weight of the supported catalyst composition.

### Comparative Example 1

### Preparation of a copper cerium alumina catalyst (5Cu10CeALU)

A portion of 10 g gamma-alumina (AX350, CRI, Ghent, Belgium), comprising particles with an average particle size between 0.2 and 0.6 mm, and with a pore volume of 0.7 ml/g, was impregnated with 7.0 ml of a solution of 3.5 g of cerium nitrate dissolved in water. After homogenizing, the product was dried at 140°C for 2 h and subsequently calcined at 550°C for 2 h while heating at a rate of 120°C/h. A portion of 1 g of the cerium-alumina was impregnated with 0.60 ml of a solution of 0.2 g of copper(II) nitrate trihydrate in water. After homogenizing, the product was dried at 120°C for 2 h and subsequently calcined at 550°C for 2 h while heating at a rate of 60°C/h. A supported catalyst was obtained, comprising 4.4% Cu, 8.4% Ce, based on the weight of the supported catalyst composition.

### Example 3

### Preparation of a copper calcium alumina catalyst (5Cu10CaALU)

A portion of 10 g alumina (AX350, CRI, Ghent, Belgium), comprising particles with an average particle size between 0.2 and 0.6 mm, and with a pore volume of 0.7 ml/g, was impregnated with 10.0 ml of a solution of 6.85 g of calcium nitrate tetrahydrate dissolved in water. After homogenizing, the product was dried at 140°C for 2 h and subsequently calcined at 550°C for 2 h while heating at a rate of 120°C/h. A portion of 1 g of the calcium-alumina was impregnated with 0.56 ml of a solution of 0.2 g of copper(II) nitrate trihydrate in water. After homogenizing, the product was dried at 120°C for 2 h and subsequently calcined at 550°C for 2 h while heating at a rate of 60°C/h. A supported catalyst was obtained, comprising 4.4% Cu, 8.3% Ca, based on the weight of the supported catalyst composition.

### Example 4

### Comparative test

The catalysts obtained in Examples 1-3 and Comparative Example 1 were tested on a parallel-reactor nanoflow equipment (Nanoflow 1A, Avantium Technologies B.V., Amsterdam, The Netherlands). An amount of 25 to 200 mg of catalyst was loaded in a reactor having an inner diameter of 4 mm. The temperature during the hydrogenation reaction was controlled by PID (Proportional Integral Differential). Experiments were performed at five temperatures, from 80 to 220°C. After reaching a temperature level the hydrogenation reaction was allowed to equilibrate for 30 to 60 minutes. The pressure during the reaction was maintained at 1 bara. A flow of acetonitrile (the partial pressure of acetonitrile was maintained at about 10 mbar) was cofed with gasflow of the hydrogen flow which was maintained at a GHSV (Gas Hourly Space Velocity) of 200 min⁻¹. After reaching equilibrium the composition of the product stream was analysed by means of ultrafast GC (Trace 2000, Interscience, Breda, The Netherlands) and MS (Airsense 2000, V&F, Absam, Austria), respectively, to monitor the selectivity of the hydrogenation reaction. The following compounds were specifically targeted for analysis: acetonitrile (AN), mono-, di-, and triethylamine (MEA, DEA, TEA, respectively), ammonia (NH₃), methane (M), and ethane (E), the latter three product occurring at high temperatures. Samples were taken at 80 minutes intervals over a period of 15 h. The test results are listed in Table 1.

**Table 1**

| Test results | | | | | | | |
|---|---|---|---|---|---|---|---|
| **Example No.** | **Catalyst** | **Temp.** | **Conv.** | **Selectivity (mol%)** | | | |
| | (code) | °C | %AN | MEA | DEA | TEA | Rest¹ |
| Ex. 1 | 5Cu5LiALU | 80 | 0 | 0 | 0 | 0 | 0 |
| " | " | 120 | 6 | 100 | 0 | 0 | 0 |
| " | " | 140 | 20 | 100 | 0 | 0 | 0 |
| " | " | 160 | 39 | 100 | 0 | 0 | 0 |
| " | " | 180 | 67 | 100 | 0 | 0 | 0 |
| " | " | 200 | 90 | 100 | 0 | 0 | 0 |
| " | " | 220 | 98 | 100 | 0 | 0 | 0 |
| " | " | 240 | 97 | 100 | 0 | 0 | 0 |
| Ex. 2 | 5Ni5LiALU | 80 | 0 | 0 | 0 | 0 | 0 |
| " | " | 120 | 8 | 82 | 18 | 0 | 0 |
| " | " | 140 | 33 | 65 | 35 | 0 | 0 |
| " | " | 160 | 70 | 69 | 31 | 0 | 0 |
| " | " | 180 | 96 | 83 | 17 | 0 | 0 |
| " | " | 200 | 99 | 99 | 0 | 0 | 1 |
| " | " | 220 | 100 | 98 | 0 | 0 | 2 |
| " | " | 240 | 98 | 88 | 0 | 0 | 11 |
| Comp.Ex. 1 | 5Cu10CeALU | 80 | 0 | 0 | 0 | 0 | 0 |
| " | " | 120 | 14 | 0 | 100 | 0 | 0 |
| " | " | 140 | 27 | 0 | 100 | 0 | 0 |
| " | " | 160 | 47 | 17 | 83 | 0 | 0 |
| " | " | 180 | 81 | 22 | 77 | 1 | 0 |
| " | " | 200 | 99 | 26 | 72 | 2 | 0 |
| " | " | 220 | 100 | 20 | 71 | 8 | 0 |
| " | " | 240 | 99 | 19 | 64 | 17 | 0 |
| Ex. 3 | 5Cu10CaALU | 100 | 0 | 0 | 0 | 0 | 0 |
| " | " | 140 | 13 | 100 | 0 | 0 | 0 |
| " | " | 180 | 72 | 100 | 0 | 0 | 0 |
| " | " | 200 | 96 | 100 | 0 | 0 | 0 |
| " | " | 220 | 99 | 100 | 0 | 0 | 0 |
| | " | 240 | 97 | 100 | 0 | 0 | 0 |

| | | | | | | | |
|---|---|---|---|---|---|---|---|
| ¹ Compounds other than MEA, DEA, and TEA, being methane, ethane, and ammonia, are denoted by "Rest". This number was calculated by subtracting the amounts of MEA, DEA, and TEA from the amount of AN. | | | | | | | |

As can be seen from Table 1, the catalysts according to the present invention show a high conversion rate of (aceto)nitrile in combination with a high selectivity of the hydrogenation of acetonitrile into monoethylamine (MEA), when using the catalyst according to Example 1, 2 and 3 at 100-240°C. At temperatures higher than 220°C, the conversion of e.g. acetonitrile is sometimes accompanied by the formation of unwanted products such as methane, ethane, and ammonia. In addition, the formation of diethylamine and triethylamine becomes more favoured at higher temperatures, so that the selectivity to the primary amine decreases. The preferred temperature range where the catalysts are used for the hydrogenation of (aceto)nitrile is 100 - 260°C, more preferably 160 - 240°C, even more preferably 180 - 220°C.

Also, it has been found that the catalysts according to the present invention provide for a high conversion rate in combination with a high selectivity when used for the hydrogenation of other nitriles such as adiponitrile, valeronitrile, succinonitrile, fatty nitriles (preferably C₆ to C₂₂), etc.

## Claims

1. Supported catalyst composition comprising:
- at least one metal selected from the group consisting of nickel, palladium, platinum, copper, silver, gold, cobalt, rhodium and iridium; and
- at least one metal selected from the group consisting of lithium, sodium, potassium, rubidium, cesium, beryllium, magnesium, calcium, strontium and barium;
wherein the composition comprises at least 1% by weight of lithium, sodium, potassium, rubidium, cesium, beryllium, magnesium, calcium, strontium or barium, or a mixture of at least two thereof, based on the weight of the supported catalyst composition.

2. Catalyst composition according to claim 1, wherein the composition comprises 4 - 20% by weight of lithium, sodium, potassium, rubidium, cesium, beryllium, magnesium, calcium, strontium or barium, or a mixture of at least two thereof, based on the weight of the supported catalyst composition.

3. Catalyst composition according to claim 1 or 2, wherein the composition comprises at least 4% by weight of lithium or calcium based on the weight of the supported catalyst composition.

4. Catalyst composition according to one or more of the preceding claims, wherein the composition comprises nickel or copper or a mixture thereof.

5. Catalyst composition according to one or more of the preceding claims, wherein the composition comprises nickel or copper and lithium.

6. Catalyst composition according to one or more of the preceding claims, wherein the composition comprises copper and calcium.

7. Catalyst composition according to one or more of the preceding claims, wherein the composition is supported on a porous support.

8. Catalyst composition according to claim 7, wherein the support comprises alumina, preferably gamma-alumina.

9. Process for the preparation of a catalyst composition,
wherein the process comprises the following steps:
(a) treating a support with a first solution containing such an amount of at least one dissolved salt of a metal selected from the group consisting of lithium, sodium, potassium, rubidium, cesium, beryllium, magnesium, calcium, strontium and barium, or a mixture of at least two thereof, that the composition obtained in step (d) comprises at least 1% by weight of lithium, sodium, potassium, rubidium, cesium, beryllium, magnesium, calcium, strontium or barium, or a mixture of at least two thereof, based on the weight of the supported catalyst composition;
(b) drying the treated support obtained in step (a);
(c) treating the dried support obtained in step (b) with a second solution containing at least one dissolved salt of a metal selected from the group consisting of nickel, palladium, platinum, copper, silver, gold, cobalt, rhodium and iridium; and
(d) drying the composition obtained in step (c).

10. Process for the preparation of a catalyst composition,
wherein the process comprises the following steps:
(x) treating a support with a first solution containing such an amount of at least one dissolved metal selected from the group consisting of lithium, sodium, potassium, rubidium, cesium, beryllium, magnesium, calcium, strontium and barium, or a mixture of at least two thereof, that the composition obtained in step (y) comprises at least 1% by weight of lithium, sodium, potassium, rubidium, cesium, beryllium, magnesium, calcium, strontium or barium, or a mixture of at least two thereof, based on the weight of the supported catalyst composition; and with a second solution containing at least one dissolved salt of a metal selected from the group consisting of nickel, palladium, platinum, copper, silver, gold, cobalt, rhodium and iridium; and
(y) drying the treated support obtained in step (x).

11. Process according to claim 9 or 10, wherein the composition is calcined after the drying steps (b) and (d) or (y).

12. Process according to claim 11, wherein the composition is calcined between 350-600°C, preferably between 500-580°C, most preferably at about 550°C.

13. Process according to one or more of the preceding claims 9-12, wherein the composition is dried in steps (b) and (d) or (y) between 80-150°C, preferably between 125-145°C, most preferably at about 140°C.

14. Process according to one or more of the preceding claims 9-13, wherein the first solution used in step (a) or (x) contains a lithium salt, preferably lithium nitrate.

15. Process according to one or more of the preceding claims 9-14, wherein the second solution used in step (c) or (x) contains a copper salt or a nickel salt or a mixture thereof, preferably copper or nickel nitrate.

16. Process according to one or more of the preceding claims 9-15, wherein the support comprises a porous support.

17. Process according to claim 16, wherein the support comprises alumina, preferably gamma-alumina.

18. Catalyst composition obtainable according to one or more of the preceding claims 9-17.

19. Use of the catalyst composition according to one or more of the preceding claims 1-8 and 18 as a hydrogenation catalyst.

20. Use according to claim 19 in the hydrogenation of nitriles.

21. Use according to claim 20 in the selective hydrogenation of nitriles for the production of primary amines.
